# EUROPÄISCHE PATENTSCHRIFT

(11) **EP 1 457 223 B1**
(45) Veröffentlichungstag und Bekanntmachung des Hinweises auf die Patenterteilung: **11.10.2006**
(21) Anmeldenummer: 03028063.0
(22) Anmeldetag: 09.12.2003
(51) Int. Cl.: A61M 16/10, A62B 9/00

(54) **Vorrichtung zur Beatmung**
Breathing device
Appareil respiratoire

(30) Priorität: 11.03.2003 DE 10310462
(43) Veröffentlichungstag der Anmeldung: 15.09.2004
(73) Patentinhaber: Weinmann Geräte für Medizin GmbH & Co. KG, 22525 Hamburg (DE)
(72) Erfinder: Feldhahn, Karl-Andreas, 22761 Hamburg (DE); Wedler, Wolfgang, 21147 Hamburg (DE); Franke, Stefan, 22547 Hamburg (DE)
(74) Vertreter: Klickow, Hans-Henning

(56) Entgegenhaltungen:
- WO-A-01/10489
- US-A- 4 016 878
- US-A- 5 482 031
- US-A- 5 537 996

## Beschreibung

Die Erfindung betrifft eine Vorrichtung zur Beatmung, die eine Atemgasversorgung sowie einen Atemgasbefeuchter aufweist, der mit einem Flüssigkeitsreservoir sowie einer Heizung versehen ist, sowie bei der die Atemgasversorgung in einem ersten Gerätemodul angeordnet ist.

Eine typische Verwendung derartiger Atemluftbefeuchter erfolgt im Zusammenhang mit Atemluftversorgungen, die im Rahmen einer CPAP-Therapie oder Bilevel-Therapie eingesetzt werden. Bei einer derartigen CPAP-Therapie (Continious-Positive-Airway-Pressure) wird einem Patienten im Zusammenhang mit der Therapie der Schlafapnoe wenigstens in Phasen eines Auftretens von Krankheitssymptomen Atemgas mit erhöhtem Druck zur Verfügung gestellt, um eine Abstützung des Atemweges zu erreichen.

Zur Vermeidung eines Austrocknens der Atemwege erweist es sich insbesondere bei längeren Beatmungsphasen als zweckmäßig, eine Befeuchtung der Atemluft durchzuführen. Derartige Befeuchtungen der Atemluft können auch bei anderen Anwendungen realisiert werden.

Typischerweise erfolgt eine Beheizung eines als Flüssigkeitsreservoir verwendeten Wassertanks des Befeuchtungssystems indirekt über einen unteren metallischen Boden des Wassertanks. Der metallische Boden wird ähnlich wie bei einem Herd auf eine Heizplatte aufgesetzt und im Bereich der Heizplatte sind elektrische Heizelemente angeordnet. Aufgrund der einzuhaltenden hygienischen Anforderungen muß der Boden zum Reinigen abnehmbar sein und trotzdem eine ausreichende Abdichtung gegen das zu erwärmende Wasser gewährleisten. Eine direkte Beheizung ist mittels eines Heizstabes direkt im Wasser möglich.

Ebenfalls ist es bereits bekannt, an ein Beatmungsgerät extern außenseitig einen Atemgasbefeuchter anzuschließen. Da die Atemgasbefeuchter in der Regel mit einer elektrischen Heizung ausgestattet sind, ist es bei derartigen Ausführungsformen aber erforderlich, außenseitig am Beatmungsgerät elektrische Anschlüsse vorzusehen. Eine derartige Ausführungsform wird beispielsweise in der PCT-WO 01/10489 beschrieben. Innerhalb eines Beatmungsgerätes angeordnete Atemgasbefeuchter werden beispielsweise in der PCT-WO 98/04311 beschrieben. Eine derartige Anordnung führt jedoch bei einem Betrieb des Beatmungsgerätes ohne Befeuchtung zu einem unnötig großen Gerätevolumen, wodurch der Benutzungskomfort reduziert wird.

Die bekannten Beatmungsgeräte werden sowohl mit fest integrierten Anfeuchtern als auch mit bei Bedarf aktivierbaren Anfeuchtungsmodulen verwendet. Bei fest eingebauten Anfeuchtern besteht der Nachteil, daß diese auch bei einer Nichtbenutzung der Anfeuchtung gereinigt werden müssen. Die Beatmungsgeräte sind darüber hinaus unnötig groß sowie schwer und der Aufwand für die Pflege und die Reinigung wird bei unbenutztem Anfeuchter unnötig hoch. Darüber hinaus wird auch die Handhabung für einen Patienten, der keine Anfeuchtung des Atemgases benötigt, unnötig kompliziert.

Bei außenseitig am Beatmungsgerät angebrachten Befeuchtungsmodulen besteht häufig das 'Problem einer ungenügenden Standfestigkeit, da bei einer häufigen Benutzung durch den Patienten selbst hohe Beanspruchungen im Hinblick auf die mechanische Festigkeit erfolgen. Darüber hinaus sind häufig elektrische Kontakte für die Übergabe der benötigten elektrischen Leistung ohne angekoppelten Anfeuchter ungeschützt und können verschmutzen.

Eine Vorrichtung zur Beatmung gemäss der Oberbegriff vom Anspruch 1 ist aus dem Dokument WO-A-01/10489 A bekannt.

Aufgabe der vorliegenden Erfindung ist es, eine Vorrichtung der einleitend genannten Art derart zu konstruieren, daß sowohl bei einer Verwendung mit aktiviertem Anfeuchter als auch ohne Anfeuchter ein verbesserter Benutzungskomfort sowie eine verbesserte Benutzungssicherheit bereitgestellt werden.

Diese Aufgabe wird erfindungsgemäß dadurch gelöst, daß das erste Gerätemodul lösbar mit einem zweiten Gerätemodul verbunden ist, daß der Atemgasbefeuchter in einem dritten Gerätemodul angeordnet ist und daß das dritte Gerätemodul mindestens bereichsweise zwischen dem ersten Gerätemodul und dem zweiten Gerätemodul anordbar ist.

Die modulare Ausbildung der Gerätekomponenten ermöglicht es, jeweils nur die Gerätekomponenten zu verwenden, die auch tatsächlich benötigt werden. Durch die Anordbarkeit des dritten Gerätemoduls mit dem Befeuchter zwischen dem ersten Gerätemodul und dem zweiten Gerätemodul ist es möglich, die jeweils benötigten elektrischen und pneumatischen Kupplungselemente in jedem Betriebszustand gegenüber von Verschmutzungen und mechanischen Beschädigungen geschützt anzuordnen. Darüber hinaus wird einem Benutzer in jedem Betriebszustand des Gerätes ein im wesentlichen gleiches äußeres Erscheinungsbild vermittelt und es erfolgt keine wesentliche Veränderung einer Anordnung oder Funktionalität von Bedienungselementen.

Zur Bereitstellung einer von einer jeweiligen Gerätekonfiguration unabhängigen Bedienoberfläche wird vorgeschlagen, daß im Bereich des zweiten Gerätemoduls ein Bedienfeld angeordnet ist.

Insbesondere ist daran gedacht, daß das Bedienfeld elektrische Bedienelemente aufweist.

Darüber hinaus ist auch vorgesehen, daß das Bedienfeld pneumatische Bedienelemente aufweist.

Eine von der jeweiligen Gerätekonfiguration unabhängige Schnittstelle zum Anschluß eines Beatmungsschlauches kann dadurch erfolgen, daß das zweite Gerätemodul ein Anschlußelement für einen verbindungsschlauch aufweist.

Eine mechanisch sehr stabile Konstruktion wird dadurch bereitgestellt, daß das zweite Gerätemodul eine schubladenförmige Halterung zur mindestens teilweisen Aufnahme des dritten Gerätemoduls aufweist.

Zur Erleichterung einer Gerätereinigung wird vorgeschlagen, daß das dritte Gerätemodul eine abnehmbare Wasserkammer aufweist.

Ein einfaches Trennen und Zusammensetzen der Gerätemodule wird dadurch unterstützt, daß das zweite Gerätemodul aus dem ersten Gerätemodul herausziehbar ausgebildet ist.

Eine weitere Konstruktionsvariante besteht darin, daß das zweite Gerätemodul aus dem ersten Gerätemodul herausnehmbar ausgebildet ist.

Ein typisches Anwendungsgebiet wird dadurch definiert, daß die Gerätemodule zur verwendung für ein CPAP- oder Bilevel-Beatmungsgerät ausgebildet sind.

Ein weiteres Anwendungsfeld besteht darin, daß die Gerätemodule zur verwendung bei einem Beatmungsgerät für invasive Anwendungen ausgebildet sind.

In den Zeichnungen sind Ausführungsbeispiele der Erfindung schematisch dargestellt. Es zeigen:
- Fig. 1: Eine perspektivische Darstellung eines Beatmungsgerätes ohne angekoppelten Vorratsbehälter für Befeuchtungsflüssigkeit,
- Fig. 2: eine schematisierte Darstellung des Gerätes nach Figur 1 mit Aufteilung in ein erstes Gerätemodul und ein zweites Gerätemodul,
- Fig. 3: die Vorrichtung gemäß Figur 2 nach einer Trennung der beiden Gerätemodule,
- Fig. 4: die vorrichtung gemäß Fig. 3 nach einem Positionieren eines dritten Gerätemoduls, das den Anfeuchter beinhaltet und
- Fig. 5: die Vorrichtung der es Figur 4 nach einem Zusammenfügen der drei Gerätemodule.

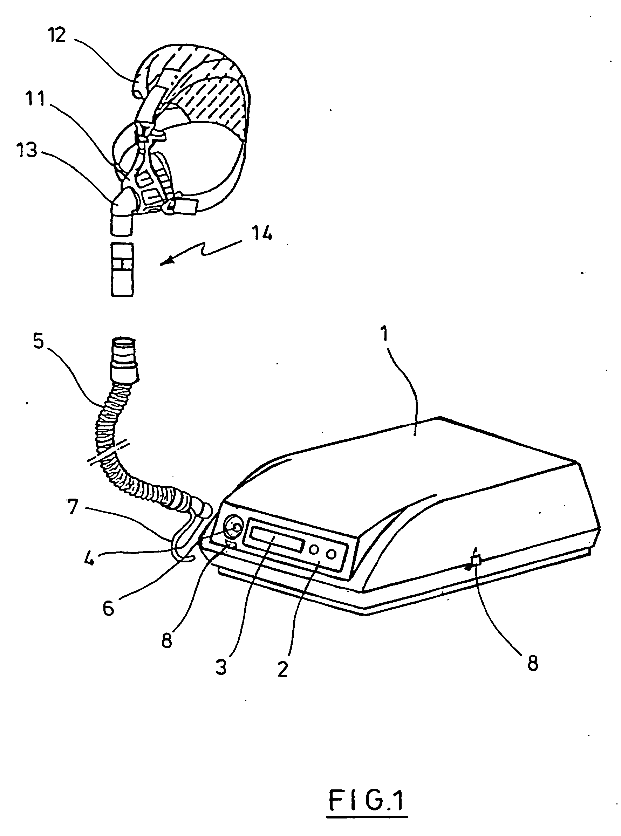

Fig. 1 zeigt den grundsätzlichen Aufbau einer Vorrichtung zur Beatmung. Im Bereich eines Beatmungsgerätes (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Die Kopplung (4) greift in einen Anschlußstutzen (6) des Beatmungsgerätes (1) ein. Entlang des Verbindungsschlauches (5) kann ein zusätzlicher Druckmeßschlauch (7) verlaufen, der über einen Druckeingangsstutzen (8) mit dem Beatmungsgerät (1) verbindbar ist. Zur Ermöglichung einer Datenübertragung weist das Beatmungsgerät (1) eine Schnittstelle (9) auf.

Im Bereich einer dem Beatmungsgerät (1) abgewandten Ausdehnung des verbindungsschlauches (5) ist ein Ausatmungselement (14) angeordnet.

Fig. 1 zeigt darüber hinaus eine Beatmungsmaske (11), die als Nasalmaske ausgebildet ist. Eine Fixierung im Bereich eines Kopfes eines Patienten kann über eine Kopfhaube (12) erfolgen. Im Bereich ihrer dem Verbindungsschlauch (5) zugewandten Ausdehnung weist die. Beatmungsmaske (11) ein Kupplungselement (13) auf.

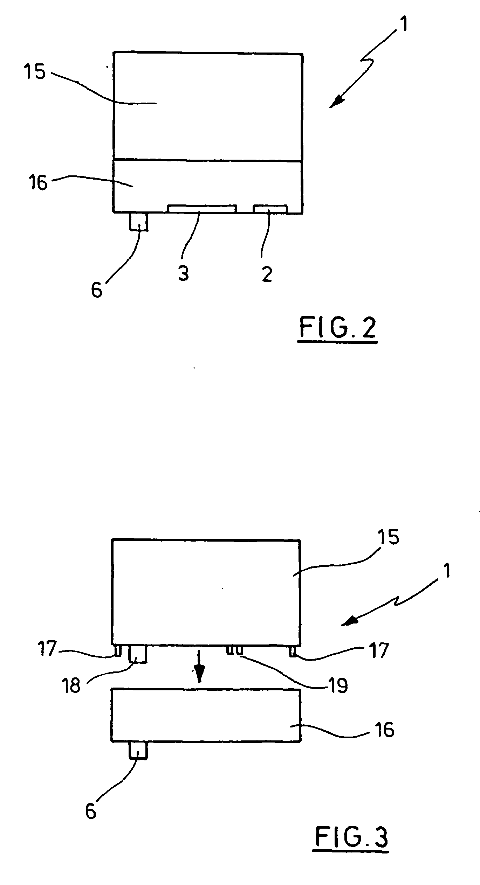

Figur 2 zeigt das Beatmungsgerät entsprechend Figur 1 in einer stark schematisierten Darstellung. Insbesondere ist zu erkennen, daß das Beatmungsgerät aus einem ersten Gerätemodul (15) sowie einem zweiten Gerätemodul (16) ausgebildet ist. Im Bereich des ersten Gerätemoduls (15) ist eine Atemgasversorgung angeordnet. Das zweite Gerätemodul (16) ist zur Aufnahme des Bedienfeldes (2) sowie der Anzeige (3) vorgesehen.

Die Darstellung in Figur 3 veranschaulicht, daß das zweite Gerätemodul (16) vom ersten Gerätemodul (15) trennbar ist. Das erste Gerätemodul (15) und das zweite Gerätemodul (16) sind über mechanische Kopplungen (17), pneumatische Kopplungen (18) sowie elektrische Kopplungen (19) miteinander verbunden. Insbesondere ist daran gedacht, die Kopplungen (17, 18, 19) ähnlich zu Stecker-Buchsen-Verbindungen auszubilden.

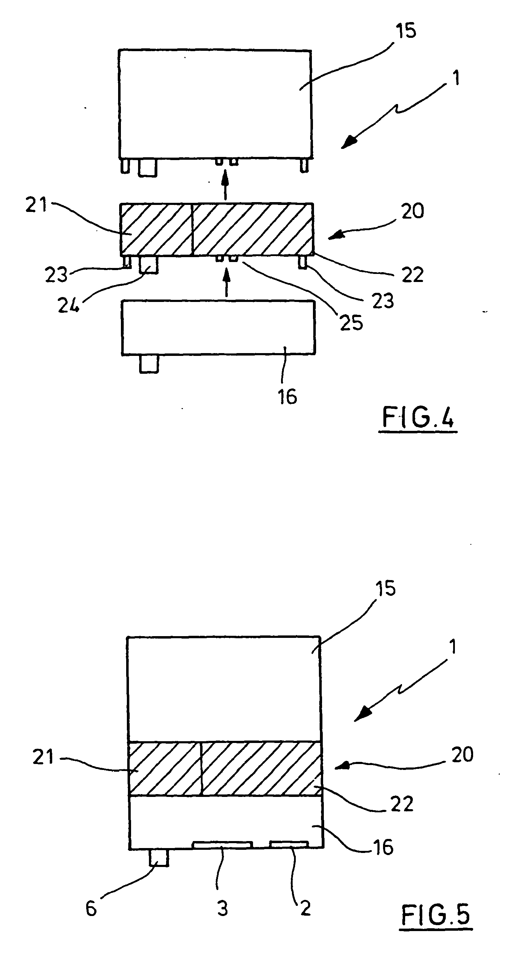

Figur 4 zeigt das Beatmungsgerät (1) nach der Positionierung eines dritten Gerätemoduls (20) zwischen dem ersten Gerätemodul (15) und dem zweiten Gerätemodul (16). Im Bereich des dritten Gerätemoduls (20) sind ein Atemgasbefeuchter (21) sowie eine Wasserkammer (22) angeordnet. Die Wasserkammer (22) ist zur Erleichterung einer Reinigungsfähigkeit vorzugsweise vom dritten Gerätemodul (20) trennbar ausgebildet.

Das dritte Gerätemodul (20) ist mit mechanischen Kopplungen (23), pneumatischen Kopplungen (24) sowie elektrischen Kopplungen (25) versehen, um eine verbindung sowohl mit dem ersten Gerätemodul (15) als auch mit dem zweiten Gerätemodul (16) durchführen zu können.. Insbesondere weist das dritte Gerätemodul (20) hierfür Verbindungselemente auf, die zu den Kopplungen (17, 18, 19) passend ausgebildet sind. Das dritte Gerätemodul (20) ist hierdurch zwischen dem ersten Gerätemodul (15) und dem zweiten Gerätemodul (16) anordbar und gewährleistet eine mechanische, pneumatische und elektrische Verbindung der Module (15, 16, 20) miteinander und untereinander.

Figur 5 zeigt die Gerätemodule (15, 16, 20) nach Figur 4 in einem miteinander verbundenen Zustand. Es ist insbesondere erkennbar, daß auch bei einer Kombination der Gerätemodule (15, 16, 20) miteinander eine äußerst kompakte Gerätekonstruktion bereitgestellt wird. Es erfolgt darüber hinaus keine Änderung im Bereich der Positionierung der Anzeige (3) sowie der Funktionalität des Bedienfeldes (2).

## Patentansprüche

1. Vorrichtung zur Beatmung, die eine Atemgasversorgung sowie einen Atemgasbefeuchter aufweist, der mit einem Flüssigkeitsreservoir und einer Heizung versehen ist, sowie bei der die Atemgasversorgung in einem ersten Gerätemodul angeordnet ist, **dadurch gekennzeichnet, daß** das erste Gerätemodul (15) lösbar mit einem zweiten Gerätemodul (16) verbunden ist, daß der Atemgasbefeuchter (21) in einem dritten Gerätemodul (20) angeordnet ist und daß das dritte Gerätemodul (20) mindestens bereichsweise zwischen dem ersten Gerätemodul (15) und dem zweiten Gerätemodul (16) anordenbar ist.

2. Vorrichtung nach Anspruch 1, **dadurch gekennzeichnet, daß** im Bereich des zweiten Gerätemoduls (15) ein Bedienfeld (2) angeordnet ist.

3. Vorrichtung nach Anspruch 1 und 2, **dadurch gekennzeichnet, daß** das Bedienfeld (2) elektrische Bedienelemente aufweist.

4. Vorrichtung nach Anspruch 1 und 2, **dadurch gekennzeichnet, daß** das Bedienfeld (2) pneumatische Bedienelemente aufweist.

5. Vorrichtung nach einem der Ansprüche 1 bis 4, **dadurch gekennzeichnet, daß** das zweite Gerätemodul (16) ein Anschlußelement für einen Verbindungsschlauch (5) aufweist.

6. Vorrichtung nach einem der Ansprüche 1 bis 5, **dadurch gekennzeichnet, daß** das zweite Gerätemodul (16) eine schubladenförmige Halterung zur mindestens teilweisen Aufnahme des dritten Gerätemoduls (20) aufweist.

7. Vorrichtung nach einem der Ansprüche 1 bis 6, **dadurch gekennzeichnet, daß** das dritte Gerätemodul (20) eine abnehmbare wasserkammer (22) aufweist.

8. Vorrichtung nach einem der Ansprüche 1 bis 7, **dadurch gekennzeichnet, daß** das zweite Gerätemodul (16) aus dem ersten Gerätemodul (15) herausziehbar ausgebildet ist.

9. Vorrichtung nach einem der Ansprüche 1 bis 7, **dadurch gekennzeichnet, daß** das zweite Gerätemodul (16) aus dem ersten Gerätemodul (15) herausnehmbar ausgebildet ist.

10. Vorrichtung nach einem der Ansprüche 1 bis 9, **dadurch gekennzeichnet, daß** die Gerätemodule (15, 16, 20) zur Verwendung für ein CPAP- oder Bilevel-Beatmungsgerät ausgebildet sind.

11. Vorrichtung nach einem der Ansprüche 1 bis 9, **dadurch gekennzeichnet, daß** die Gerätemodule (15, 16, 20) zur verwendung bei einem Beatmungsgerät für invasive Anwendungen ausgebildet sind.

## Claims

1. Respiratory device with a respiratory gas supply and a respiratory gas humidifier, which is provided with a liquid reservoir and a heating system, the respiratory gas supply being disposed in a first device module, **characterised in that** the first device module (15) is releasably connected to a second device module (16), the respiratory gas humidifier (21) is disposed in a third device module (20) and at least certain regions of the third device module (20) can be disposed between the first device module (15) and the second device module (16).

2. Device as claimed in claim 1, **characterised in that** a control panel (2) is disposed in the region of the second device module (15).

3. Device as claimed in claim 1 and 2, **characterised in that** the control panel (2) has electric control elements.

4. Device as claimed in claim 1 and 2, **characterised in that** the control panel (2) has pneumatic control elements.

5. Device as claimed in one of claims 1 to 4, **characterised in that** the second device module (16) has a connector element for a connecting flexible pipe (5).

6. Device as claimed in one of clams 1 to 5, **characterised in that** the second device module (16) has a drawer-shaped retaining means for at least partially accommodating the third device module (20).

7. Device as claimed in one of claims 1 to 6, **characterised in that** the third device module (20) has a removable water chamber (22).

8. Device as claimed in one of claims 1 to 7, **characterised in that** the second device module (16) can be pulled out of the first device module (15).

9. Device as claimed in one of claims 1 to 7, **characterised in that** the second device module (16) is designed so that it can be removed from the first device module (15).

10. Device as claimed in one of claims 1 to 9, **characterised in that** the device modules (15, 16, 20) are designed for use with a CPAP or bi-level respiratory device.

11. Device as claimed in one of claims 1 to 9, **characterised in that** the device modules (15, 16, 20) are designed for use with a respiratory device for invasive applications.

## Revendications

1. Appareil respiratoire qui comprend une alimentation en gaz respiratoire, de même qu'un humidificateur de gaz respiratoire, pourvu d'un réservoir à fluide et d'un chauffage, et dans lequel l'alimentation en gaz respiratoire est agencée dans un premier module d'installation, **caractérisé en ce que** le premier module d'installation (15) est relié de façon détachable à un second module d'installation (16), **en ce que** l'humidificateur de gaz respiratoire (21) est agencé dans un troisième module d'installation (20) et **en ce que** le troisième module d'installation (20) peut être agencé au moins en partie entre le premier module d'installation (15) et le second module d'installation (16).

2. Dispositif selon la revendication 1, **caractérisé en ce qu'**un tableau de commande (2) est agencé dans la région du deuxième module d'installation (16).

3. Dispositif selon les revendications 1 et 2, **caractérisé en ce que** le tableau de commande (2) présente des éléments de commande électriques.

4. Dispositif selon les revendications 1 et 2, **caractérisé en ce que** le tableau de commande (2) présente des éléments de commande pneumatiques.

5. Dispositif selon l'une des revendications 1 à 4, **caractérisé en ce que** le second module d'installation (16) comprend un élément de raccordement pour un tuyau de liaison souple (5).

6. Dispositif selon l'une des revendications 1 à 5, **caractérisé en ce que** le second module d'installation (16) comprend un support en tiroir pour recevoir au moins partiellement le troisième module d'installation (20).

7. Dispositif selon l'une des revendications 1 à 6, **caractérisé en ce que** le troisième module d'installation (20) comprend une chambre à eau (22) amovible.

8. Dispositif selon l'une des revendications 1 à 7, **caractérisé en ce que** le second module d'installation (16) peut être extrait du premier module d'installation (15).

9. Dispositif selon l'une des revendications 1 à 7, **caractérisé en ce que** le second module d'installation (16) peut être démonté du premier module d'installation (15).

10. Dispositif selon l'une des revendications 1 à 9, **caractérisé en ce que** les modules d'installation (15, 16, 20) sont réalisés pour un appareil respiratoire à pression positive continue ou à bi-niveau.

11. Dispositif selon l'une des revendications 1 à 9, **caractérisé en ce que** les modules d'installation (15, 16, 20) sont réalisés pour un appareil respiratoire pour des usages invasifs.
